# EUROPEAN PATENT SPECIFICATION

(11) **EP 4 176 722 B1**
(45) Date of publication and mention of the grant of the patent: **21.10.2026**
(21) Application number: 22461629.2
(22) Date of filing: 07.11.2022
(51) Int. Cl.: A01N 59/16, A01N 25/04, A01N 25/12, A01P 1/00

(54) **AGED ZERO-VALENT IRON FOR USE AS AN ANTIVIRAL AGENT AND A METHOD FOR THE PREPARATION OF ZERO-VALENT IRON**
GEALTERTES NULLWERTIGES EISEN ZUR VERWENDUNG ALS ANTIVIRALES MITTEL UND VERFAHREN ZUR HERSTELLUNG VON NULLWERTIGEM EISEN
FER À VALENCE ZÉRO VIEILLI POUR UNE UTILISATION EN TANT QU'AGENT ANTIVIRAL ET PROCÉDÉ DE PRÉPARATION DE FER À VALENCE ZÉRO

(30) Priority: 08.11.2021 PL 43945621
(43) Date of publication of application: 10.05.2023
(73) Proprietor: Instytut Chemii Bioorganicznej Polskiej Akademii Nauk, 61-704 Poznan (PL)
(72) Inventor: KIERZEK, Elzbieta, Poznan (PL); WIECZOREK, Klaudia, Skórzewo (PL); FOLTYNOWICZ, Zenon, Poznan (PL); MUC, Karol, Poznan (PL)
(74) Representative: Kawczynska, Marta Joanna

(56) References cited:
- WO-A1-2021/206634
- US-A1- 2007 256 985
- KIM JEE YEON ET AL: "Inactivation of MS2 Coliphage by Ferrous Ion and Zero-Valent Iron Nanoparticles", ENVIRONMENTAL SCIENCE & TECHNOLOGY, vol. 45, no. 16, 20 July 2011 (2011-07-20), US, pages 6978 - 6984, XP093031676, ISSN: 0013-936X, DOI: 10.1021/es201345y
- SHEARER ADRIENNE E.H. ET AL: "Enhanced Removal of Norovirus Surrogates, Murine Norovirus and Tulane Virus, from Aqueous Systems by Zero-Valent Iron", vol. 81, no. 9, 1 September 2018 (2018-09-01), US, pages 1432 - 1438, XP093031788, ISSN: 0362-028X, Retrieved from the Internet <URL:https://dul.usage.elsevier.com/doi/> DOI: 10.4315/0362-028X.JFP-18-054
- CHUNJIAN SHI ET AL: "Removal of viruses and bacteriophages from drinking water using zero-valent iron", SEPARATION AND PURIFICATION TECHNOLOGY, vol. 84, 2 July 2011 (2011-07-02), pages 72 - 78, XP028392284, ISSN: 1383-5866, [retrieved on 20110702], DOI: 10.1016/J.SEPPUR.2011.06.036
- YOU YOUWEN ET AL: "Removal and Inactivation of Waterborne Viruses Using Zerovalent Iron", vol. 39, no. 23, 2 November 2005 (2005-11-02), US, pages 9263 - 9269, XP093031771, ISSN: 0013-936X, Retrieved from the Internet <URL:https://pubs.acs.org/doi/pdf/10.1021/es050829j> DOI: 10.1021/es050829j
- CHOPYK JESSICA ET AL: "Zero-valent iron sand filtration reduces concentrations of virus-like particles and modifies virome community composition in reclaimed water used for agricultural irrigation", vol. 12, no. 1, 11 April 2019 (2019-04-11), XP093031799, Retrieved from the Internet <URL:http://link.springer.com/article/10.1186/s13104-019-4251-y/fulltext.html> DOI: 10.1186/s13104-019-4251-y
- NIKKI GOLDSTEIN ET AL: "Influence of synthesis parameters on iron nanoparticle size and zeta potential", JOURNAL OF NANOPARTICLE RESEARCH ; AN INTERDISCIPLINARY FORUM FOR NANOSCALE SCIENCE AND TECHNOLOGY, KLUWER ACADEMIC PUBLISHERS, DO, vol. 14, no. 4, 6 March 2012 (2012-03-06), pages 1 - 15, XP035083415, ISSN: 1572-896X, DOI: 10.1007/S11051-012-0760-5
- LEE CHANGHA ET AL: "Bactericidal Effect of Zero-Valent Iron Nanoparticles on Escherichia coli", vol. 42, no. 13, 1 July 2008 (2008-07-01), US, pages 4927 - 4933, XP093032132, ISSN: 0013-936X, Retrieved from the Internet <URL:https://www.ncbi.nlm.nih.gov/pmc/articles/PMC2536719/pdf/nihms47981.pdf> DOI: 10.1021/es800408u
- HAHNEMÜHLE: "Industry & Laboratory | Product Profile & Application", FILTER PAPERS & MEMBRANES, 21 March 2018 (2018-03-21), pages 1 - 144, XP093032275, Retrieved from the Internet <URL:https://www.hahnemuehle.com/fileadmin/user_upload/bilder/Filtration/pdf/web-10603856_Katalog_Filtration_EN.pdf> [retrieved on 20230316]

## Description

### Field of invention

The invention is directed to the use of the zero-valent iron as an antiviral agent, in particular for the prevention and/or control of viral infection, especially caused by a virus of the *Orthomyxoviridae* family, such as the influenza virus, in particular the influenza type A virus, as well as for use as a disinfectant. The invention is also directed to a method for the preparation of the zero-valent iron obtained in a reduction reaction of an iron salt with sodium tetraborohydride, and the zero-valent iron prepared using this method for use as an antiviral agent, in particular in the prevention and/or control of viral infection, especially caused by a virus of the *Orthomyxoviridae* family, such as the influenza virus, in particular influenza type A virus.

### Background of the invention

Viruses of the *Orthomyxoviridae* family are a group of enveloped viruses that includes a number of viruses whose representative example is the influenza virus. Such viruses having a continuously mutating genome change very frequently their properties, such as they exceptionally rapidly acquire resistance to available antiviral medicines and vaccines, which, as known from history, can constitute a highly fatal and rapidly spreading hazard. Therefore, there is an urgent need to develop new effective antiviral agents protecting against infection due to viruses of this kind, including to control infection caused by viruses of the *Orthomyxoviridae* family, such as the influenza virus, in particular influenza type A virus. Nanotechnology is one of the youngest and simultaneously most dynamically growing fields of science, and interest in nanotechnology has increased significantly in recent years. Its development has been possible due to new technologies as well as the continuously increasing demand for novel medicines and therapies. In addition, the possibility of synthesizing nanoparticles having various shapes, sizes or modifications of their surface distinguishes their immense application potential.

Nanoparticles are most frequently used as carriers for substances of various kinds: proteins, nucleic acids or medicines. Various application strategies for nanoparticles to control viruses are known, such as the use of nanoparticles as vaccine carriers, gene expression inhibitors or medicines. Moreover, metal nanoparticles are increasingly more frequently used not only as carriers, but also as antiviral compounds. Inhibitory properties against the proliferation of the influenza virus show, *inter alia,* such nanoparticles as silver, gold, zinc oxide, iron oxide, titanium dioxide, calcium oxide and zirconium dioxide nanoparticles [1]. It has already been found that iron oxide (Fe₃O₄) nanoparticles used in a concentration of 30 µg/mL inhibit RNA synthesis of the A H1N1 influenza virus in MDCK cells. The results suggest that the antiviral properties result from the binding of viral RNA to the nanoparticles [2]. A different study described the antiviral activity of iron oxide (Fe₃O₄) nanoparticles synthesized in one stage in a solvothermal system by combining FeCl₃ and sodium acetate in ethylene glycol. The nanoparticles have the ability to peroxidize lipids of the viral envelope, which in turn results in the inactivation of the influenza type A virus. The presented studies show that the iron oxide nanoparticles are effective inhibitors against 12 subtypes of the influenza type A virus. In addition, the authors of the report suggest that iron oxide nanoparticles may be a general antiviral agent against enveloped viruses, such as HIV, Ebola virus, Zika virus, West Nile virus and Nipah virus [3].

In the literature, the application of nanoiron iron nanoparticles as a part of antiviral filtration membrane against enveloped and non-enveloped viruses has also been reported. Within this approach, amyloid nanofibers were manufactured and coated with iron oxyhydroxide nanoparticles. The iron oxyhydroxide nanoparticles were precipitated directly on the nanofibers by increasing the pH value in the presence of FeCl₃·6H₂O. Studies were carried out on three enveloped viruses: bacteriophage Φ6, influenza A H1N1 virus and the SARS CoV-2 virus, as well as with two non-enveloped viruses: bacteriophage MS2 and enterovirus 71. Except for enterovirus 71, filtration using iron oxide nanoparticles lowered the viral titer below the detection limit for all the viruses studied. In addition, the titer of enterovirus 71 decreased to 1/3 of the initial value [4]. However, the studies carried out did not explain the action of the resulting filter. It was found that the antiviral activity was neither caused by the retention of virions on the filtration material due to the small pore size nor by the viral titer decreased as a result of contact with the iron nanoparticles.

Considering their reducing and catalytic properties, iron nanoparticles in the form of zero-valent iron nanoparticles (nZVIs) are considered the most important nanoparticles allowing the effective reduction of a wide range of environmental pollutants, both of organic and inorganic origin [5]. Zero-valent iron (ZVI) has been used for the purification of polluted groundwater for almost two decades [6]. Recent results have shown that ZVI is also effective in removing important contaminants from drinking water, including viruses and bacteria [7] or chemical and ionic contaminants [6,8,9]. It should be noted that zero-valent iron nanoparticles have not been used so far as antiviral agents against infection caused by a virus of the *Orthomyxoviridae* family. However, their activity has been reported against the following viruses: human and murine norovirus [10], bacteriophage MS-2 [11-13] and ΦX-174 [12,13], adenovirus 41 and Aichi virus [12] as well as virus-like particles [14]. Furthermore, nZVIs oxidize in water and "age" over time, which leads to the formation of non-toxic magnetite and/or maghemite [15,16], and this suggests a low risk of toxicity toward ecosystems.

WO 2021/206634 A1 discloses an antiviral activity of surface oxidized zero valent iron particles having size 1-800 µm against H3N2 virus. The surface of the zero valent iron particles is completely oxidated so a zero valent iron is not present on the active surface and cannot act as an antiviral agent.

NIKKI GOLDSTEIN ET AL: "Influence of synthesis parameters on iron nanoparticle size andzeta potential', JOURNAL OF NANOPARTICLE RESEARCH; AN INTERDISCIPLINARY FORUM FOR NANOSCALE SCIENCE AND TECHNOLOGY, vol. 14, no. 4, (2012-03-06), pages 1-15, discloses a method of preparing ZVI nanoparticles by reducing ferrous sulfate with different FeSO₄/NaBH₄ molar ratios (0.5, 1, 2, 4, 8) in the presence of ATMP or CMC as suspension stabilisers under argon atmosphere producing particles of different sizes. The particles were separated by certifugation and resuspended in e.g. water. It is disclosed that without stabilizers the particles will agglomerate, however no nylon filter was used.

US 2007/256985 Aldiscloses a method of preparing ZVI nanoparticles by reducing ferrous sulfate with NaBH₄ at FeSO₄/NaBH₄ molar ratio 1:2 under nitrogen atmosphere in the presence and absence of CMC suspension stabiliser. Stabilised nanoparticles have average size of 11.2 nm. Non-stabilised nanoparticles are agglomerating which increases their size (not given) and reduces surface area and catalytic activity. No nylon filter was used in the method. CHANGHA LEE ET AL: "Bactericidal Effect of Zero-Valent Iron Nanoparticles on Escherichia coli", ENVIRONMENTAL SCIENCE & TECHNOLOGY, vol. 42, no. 13, (2008-07-01), pages 4927-4933, discloses the synthesis of the ZVI nanoparticles by reducing FeSO₄ with NaBH₄ at the molar ratio 1:1.5, centrifuging, decanting and washing 3 times with 0.1 mM (pH =4) HCI (centrifuging and decanting after each wash, these washing remove sulfate and borate anions), all the steps under N₂ atmosphere (the particles form linear agglomerates, the estimated average agglomerate diameter being between 600-800 nm).The suspension was centrifuged and decanted but not filtered.

Among the practical applications of zero-valent iron nanoparticles, their use as active ingredients of packaging containing so-called oxygen scavengers is known ([17] and patent specifications PL227096 and PL227585).

In addition, the use of zero-valent iron nanoparticles is known from the patent application P.432267 as an ingredient of a polymer matrix applied in the form of a thin coating on the internal layer of a packaging material having antibacterial properties as well as activity against yeasts and molds. By incorporation of zero-valent iron nanoparticles into a nanocomposite coating in the disclosed solution, inhibition of the growth of microorganisms, such as Grampositive and Gram-negative bacteria, yeasts and molds, was achieved. However, no studies have yet been performed to verify the antiviral activity of such nanoparticles.

Methods for the preparation of metal nanoparticles are known, and one of the commonly used methods is the chemical reduction of metal salt in solution.

Methods for the preparation of zero-valent iron nanoparticles and their applications are disclosed in patent specifications PL227096 and PL227585.

In the specification PL227585, a method for the preparation of boron-doped zero-valent iron nanoparticles in a reduction reaction of an iron(III) salt with sodium tetraborohydride in precisely defined reaction conditions, such as the molar ratio of the iron salt to NaBH₄ is 1:3, was disclosed. As a result of the method carried out in this way, zero-valent iron nanoparticles were obtained having exceptionally high oxygen binding activity in any environment, including anhydrous, and nanoparticles obtained in this way were used as an oxygen scavenging material to be placed inside packages to eliminate oxygen from the packages as well as to capture oxygen penetrating inside the packaging material.

A similar method for the preparation of zero-valent iron nanoparticles, again using a reduction reaction of an iron salt with sodium tetraborohydride under conditions such that the molar ratio of the iron salt to NaBH₄ is 1:3, was disclosed in the specification PL227096. It was also stated in the specification that the nanoparticles obtained using the developed method had oxygenbinding properties and could be used as an oxygen scavenger in packages.

Methods for the preparation of zero-valent iron nanoparticles disclosed in the foregoing patent specifications are expensive due to the required large amount of the reducing agent consumed in the reduction reaction. In addition, a large amount of hydrogen gas is released as a result of the reaction carried out in this way, which is dangerous.

Therefore, there is a continuous demand in the art for new and effective antiviral agents, especially for the prevention and/or control of viral infection, in particular infection caused by viruses of the *Orthomyxoviridae* family, such as the influenza virus, especially the influenza type A virus. In addition, there is also continuous demand for new and effective methods for the preparation of zero-valent iron, in particular in the form of nanoparticles.

The objective of the invention is to develop new and effective antiviral agents for the prevention and/or control of viral infection, especially caused by a virus of the *Orthomyxoviridae* family, such as the influenza virus, in particular the influenza type A virus, and a method for the preparation thereof, without the disadvantages known in the prior art.

These objectives have been achieved by the inventions defined in the attached patent claims, being part of this application, and illustrated in detail in this specification.

### Brief description of the invention

The present invention provides the zero-valent iron for use as an antiviral agent.

The said zero-valent iron nanoparticles have sizes of <100 nm and form agglomerates having an average hydrodynamic size within a range of 250 to 900 nm and were subjected to aging by exposure to air at room temperature, wherein aging conditions were selected so that no more than 1% of the formulation thereof underwent surface oxidation.

The said zero-valent iron is preferably for use in the prevention and/or control of viral infection.

The virus is preferably a virus of the *Orthomyxoviridae* family, in particular the influenza virus, especially the influenza type A virus.

The said zero-valent iron is preferably used as a disinfectant.

The invention provides also a method for the preparation of zero-valent iron nanoparticles in a reduction reaction of an iron salt with sodium tetraborohydride NaBH₄, which consists in that an iron(II) salt or a mixture of iron(II) salts used as a starting substance for a reduction in a molar ratio of the iron(II) salt to NaBH₄ of 1:2. The reaction is carried out in a protective atmosphere of inert gas until precipitate forms, after precipitation the precipitate is subsequently decanted and washed until complete removal of anions of the salt used for the reduction and the precipitate is then filtered using a Nylon filter and dried.

Iron(II) chloride is preferably used as the starting substance.

A mixture of iron(II) chloride and iron(II) sulfate is preferably used as the starting substance.

A Nylon fabric filter is preferably used, in particular with a 20 DEN weave.

The drying is preferably carried out through freeze-drying.

The zero-valent iron after the drying stage is preferably subjected to aging.

The aging is more preferably carried out until no more than 1% of the surface is oxidized.

### Detailed description of the invention

Zero-valent iron (ZVI) is iron in the zero oxidation state, that is, with zero valency.

According to the definition recommended by the International Union of Pure and Applied Chemistry (IUPAC), nanoparticles are particles of any shape and approximately having one of the dimensions in a range from 10⁻⁹ to 10⁻⁷ m. The primary feature of nanoscale materials and particles is that they have different properties than on a larger scale. Furthermore, due to the possibility of selecting various sizes, morphology and modification of nanoparticle surfaces, they are even more desirable for broader applications. Therefore, nanoparticles have a specific geometric structure having a high surface-to-volume ratio, wherein the shape of the structure influences properties.

Zero-valent iron nanoparticles may form agglomerates having larger sizes than the nanometer scale. According to the invention, the average hydrodynamic size of such agglomerates is within a range of 250 to 900 nm.

The zero-valent iron may be prepared using various methods known in the art, including through synthesis with chemical reduction, wherein it may be preferably prepared using the method according to the invention as described below and illustrated in the Examples.

In the developed method for the preparation of the zero-valent iron according to the invention, ZVI is obtained using a method that consists in a reduction reaction of an iron salt with sodium tetraborohydride NaBH₄, wherein an iron(II) salt or a mixture of iron(II) salts is used as a starting material, preferably iron(II) chloride [FeCl₂] or a mixture of iron(II) chloride and iron(II) sulfate [FeCl₂+ FeSO₄].

The reduction reaction proceeds according to the following scheme:

FeCl₂ + 2NaBH₄ + 6H₂O → Fe°↓ + 2H₃BO₃ + 2NaCl + 7H₂↑

The method for the preparation of ZVI according to the invention may be carried out, for example, as follows.

The method is carried out in a glass flask having a volume corresponding to the amount of aqueous solutions of the reagents being used, fitted with a gas inlet and outlet. In the synthesis, 0.5 M FeCl₂ solution (obtained by dissolving 63.5 g FeCl₂ in 1000 mL degassed distilled water) and 0.5 M NaBH₄ solution (prepared by dissolving NaBH₄ in degassed distilled water) are used. Subsequently, the NaBH₄ solution is added dropwise at a rate of 0.25-0.3 mL/s to the iron chloride solution until no further formation of black precipitate and release of hydrogen occurs. The reaction is carried out in a protective atmosphere of inert gas, preferably argon. The reaction mixture is left to stand for 24 h, and the remaining sodium borohydride solution is added in portions (50 mL each) and left to stand for 24 h to stabilize the precipitate. After decanting, the ZVI precipitate is washed with distilled water until the Cl⁻ ions were completely removed. Subsequently, the precipitate is filtered using a Nylon filter, preferably made of Nylon fabric with a 20 DEN weave, developed by the inventors of the present invention. The filter allows particularly effective separation of zero-valent iron nanoparticles from the solution, which the separation is a significant problem in the methods known in the prior art. The filtered precipitate is dried, preferably through freeze-drying. Such a filtration and drying procedure provides homogeneity of the composition of the obtained ZVI formulation. The obtained ZVI formulation is stored in a sealed container in a protective atmosphere of inert gas, for example argon.

According to the invention, the iron(II) chloride used as the starting material in the method for the preparation of ZVI is a more expensive reagent compared to the previously used iron(III) chloride, but in the reduction reaction thereof less sodium borohydride is used (per 1 g ZVI, 1.7 g NaBH₄ and 2.3 g FeCl₂, is required, while to obtain 1 g ZVI in the reduction reaction of iron(III) chloride, 2.3 g NaBH₄ and 2.9 g FeCl₂ needs to be used). This translates into a more economic effect of the method carried out according to the invention. In addition, in the reduction reaction carried out according to the developed method for the preparation of zerovalent iron, much less hydrogen gas is released (7H₂↑ compared to 10.5H₂↑ in the conventional method), which is dangerous, and this is observed in the methods known in the prior art. Therefore, the method for the preparation of ZVI disclosed herein provides measurable benefits in that the method is more economical and less harmful.

The inventors of the present invention unexpectedly concluded based on the studies carried out that the aged zero-valent iron nanoparticles agglomerates as described above, in particular prepared according to the developed method, has potent antiviral activity.

The conditions of ZVI synthesis (ratio of the amount of the iron salt to the borohydride; process for washing and drying the precipitate) and subsequent treatment (storage in a protective atmosphere of inert gas and/or aging) affect the sizes of the resulting particles and their reactivity [17-22].

The inventors of the present invention carried out studies of ZVI in terms of its antiviral properties and performed a number of assays for representative viral strains of the *Orthomyxoviridae* family, including the influenza virus, to determine the effect of incubating virions with ZVI on the amount and infectivity of viral particles. It was found as a result that ZVI significantly reduced the amount of the virus.

To determine the titer of the influenza virus, a highly sensitive immunofluorescence assay was performed to examine the number of viral infectious units in one milliliter of the tested viral supernatant. It was found that when ZVI with a concentration of 3 mg/mL was used, the viral titer significantly decreased as early as after 15 minutes of incubation with the influenza virus. In addition, already the lowest concentration of the ZVI used for studies (i.e., 0.75 mg/mL) showed a significant decrease in the influenza virus titer after two hours of incubation with the virus. Furthermore, it was observed that with increasing concentrations of ZVI, the efficacy of inactivation of the influenza virus increased. At the highest ZVI concentration used (8 mg/mL), the influenza virus titer decreased dramatically from 7.2 log₁₀ to 2.7 log₁₀, that is, as much as 99.99% of the amount of the virus. Therefore, the antiviral activity of ZVI was exceptionally potent and concentration-dependent. In addition, a Western Blot analysis of viral proteins was performed. It showed that the binding of antibodies targeted to influenza virus proteins was not possible after influenza virus incubation with the lowest ZVI concentration, even though their presence on the membrane was confirmed using a different technique, which indicates the inactivation of influenza virus protein antigens.

The results of studies carried out by the inventors of the present invention on selected strains of the influenza type A virus, being a representative virus of the *Orthomyxoviridae* family, showed effective antiviral activity of the zero-valent iron.

In addition, antiviral activity studies also investigated the effect of the aging time of zero-valent iron on its activity.

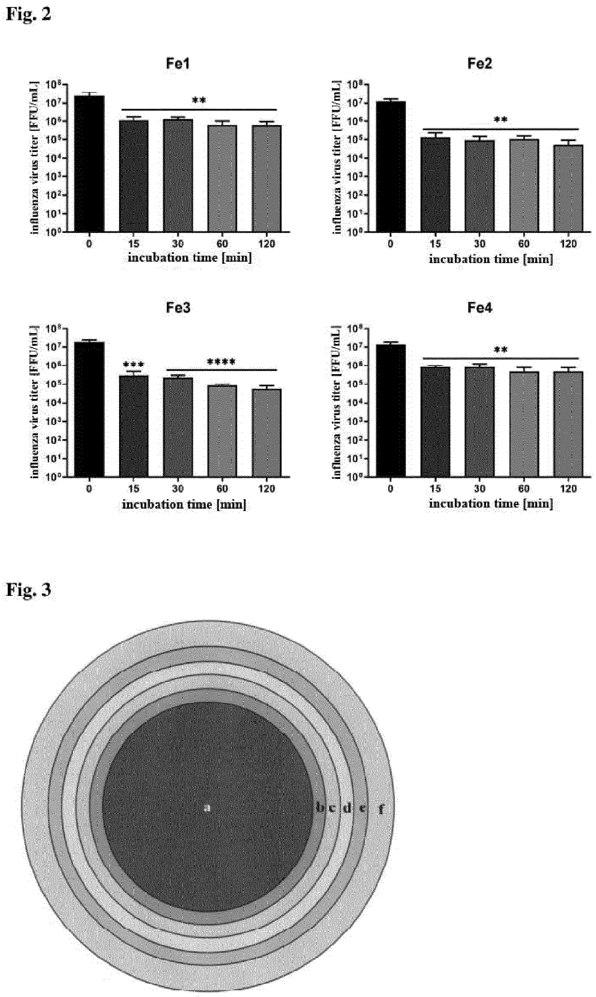

According to the invention, the aging of ZVI is understood as its exposure to air at room temperature until its color changes from black to brown, typically for 1 h. Aging conditions are selected so that no more than 1% of the formulation undergoes surface oxidation. The structure of an aged ZVI nanoparticle of the core-passivation coating type is schematically illustrated in Fig. 3, which differs from the so-called "fresh ZVI" in that a layer of oxides at different oxidation states forms on the surface, and the layer is important for the antiviral activity of ZVI, as discussed below and illustrated in the following examples of the invention.

An advantageous effect of ZVI aging on its antiviral activity was shown by the studies carried out.

The obtained study results of the effect of ZVI aging time on its antiviral activity clearly show that partially oxidized ZVI formulations have considerably higher antiviral activity. At a concentration of 1 mg/mL, freshly prepared ZVI in PBS buffer results in the viral titer decrease by 1 log₂, while ZVI after 36 h of aging in PBS buffer results in the influenza viral titer decrease by 2 log₂. The viral titer decreases to zero when 2.5 mg/mL freshly prepared ZVI is used, whereas the same effect may be obtained using a lower amount of a ZVI formulation aged for 36 h in PBS, namely 1.75 mg/mL of aged ZVI is sufficient to decrease the viral titer to zero. As described above and illustrated in the Examples with a representative example of a virus of the *Orthomyxoviridae* family, that is, influenza type A virus, the zero-valent iron used in the present invention, especially prepared using the method according to the invention, shows potent antiviral properties, especially against viruses of the *Orthomyxoviridae* family. Due to its properties, zero-valent iron nanoparticle agglomerates described above may therefore be used in a number of biological and biomedical applications, including, without limitation, for the control and prevention of infections caused by a virus of the *Orthomyxoviridae* family as well as for other enveloped viruses due to their structural similarity to a virus of the *Orthomyxoviridae* family.

The present invention will be illustrated below by way of examples and figures which however are not intended to limit, in any way, the scope of protection of the invention as defined in the claims.

### Brief description of the figures

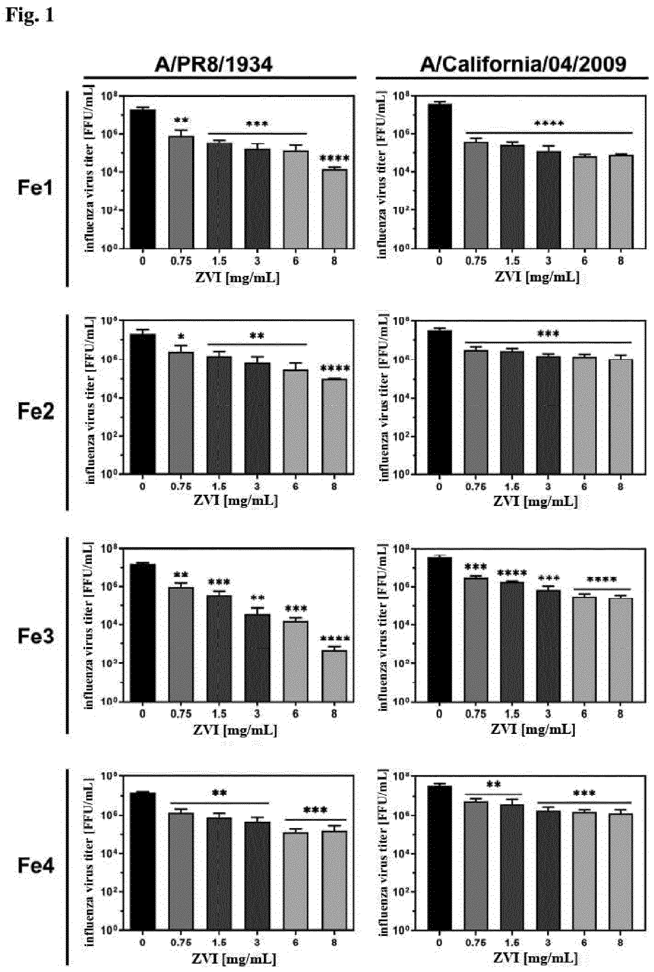

Fig. 1 presents charts illustrating the study results of the effect of ZVI formulations Fe1 - Fe4 obtained according to Examples 1a - 1d on the decrease of the influenza virus titer after 2 hours of incubation. The number of influenza virus infectious units was determined using an immunofluorescence assay for A/California/04/2009 (H1N1) and A/PR8/1934 (H1N1) strains. The data are expressed as a mean ± standard deviation from three independent experiments, each performed in triplicate. Student's two-tailed distribution unpaired t-test was performed for statistical comparison (*p < 0.05, **p < 0.01, ***p < 0.001, ****p < 0.0001). Specific values are shown in Table 2 (Fe1), Table 3 (Fe2), Table 4 (Fe3) and Table 5 (Fe4).
Fig. 2 presents charts illustrating the study results of the effect of ZVI formulations Fe1 - Fe4 obtained according to Examples 1a - 1d on the decrease of the influenza virus titer after incubation, studied in time intervals of 15, 30, 60 and 120 minutes. The study was carried out for a ZVI concentration of 3 mg/mL. The number of influenza virus infectious units was determined using an immunofluorescence assay for strain A/PR8/1934 (H1N1). The data are expressed as a mean ± standard deviation for three independent experiments, each performed in triplicate. Student's two-tailed distribution unpaired t-test was performed for statistical comparison (*p < 0.05, **p < 0.01, ***p < 0.001, ****p < 0.0001). Precise values are shown in Table 2 (Fe1), Table 3 (Fe2), Table 4 (Fe3) and Table 5 (Fe4).
Fig. 3 presents schematically a structure of an aged ZVI nanoparticle of the core-oxide passivation coating type: a) zero-valent iron core, αFe(0), b) FeO, c) Fe(OH)₂, d) FeO·Fe₂O₃, e) FeOOH, f) Fe₂O₃·0.5H₂O.
Fig. 4 presents an electron microscope photograph of ZVI formulation Fe1.
Fig. 5 presents an electron microscope photograph of ZVI formulation Fe2.

### Examples

### Preparation of zero-valent iron (ZVI) and aged ZVI and study of their antiviral activity

All the procedures and analyses presented below were performed using commercially available test kits, reagents and equipment, by following the instructions of the manufacturers of the used kits, reagents and equipment, unless otherwise stated in the description. All the analyses were performed using standard, generally known methods used in the art to which the present invention belongs.

### Example 1. General description of the preparation of zero-valent iron (ZVI) in a reduction reaction of iron(II) chloride with sodium borohydride

The method was carried out in a glass flask having a volume of 5 dm³ fitted with a gas inlet and outlet. 63.5 g FeCl₂ was dissolved in 1000 mL of degassed distilled water and 0.5 M solution was obtained. 2500 mL of 0.5 M (in degassed distilled water) NaBH₄ solution was prepared separately, and it was subsequently added dropwise at a rate of 0.25-0.3 mL/s to the iron chloride solution until no further formation of black precipitate and release of hydrogen occurred, typically 2000 mL. The reaction was carried out in a protective atmosphere of inert argon gas. The reaction mixture was left to stand for 24 h, and the remaining 500 mL of the borohydride solution was added in portions (50 mL each) and left to stand for 24 h to stabilize the precipitate. After decanting, the precipitate was washed with distilled water until the Cl⁻ ions were completely removed. Subsequently, the precipitate was filtered using a Nylon fabric filter with a 20 DEN weave and dried by freeze-drying. The resulting ZVI formulation was stored in a sealed container in a protective atmosphere of inert argon gas for further use.

### Example 1a. ZVI formulation Fe1

A batch of the ZVI formulation as obtained in Example 1 was subjected to aging through exposure to air at room temperature for 1 h until the color of the formulation changed from black to brown.

Aging conditions were selected so that no more than 1% of the formulation underwent surface oxidation. Therefore, 10 g ZVI was placed in a glass container with a volume of 150 mL filled with air and it was tightly sealed.

In an oxidation reaction, 1 g Fe may bind 300 mL of O₂. A container with a volume of 150 mL filled with air (~ 10 mL for the formulation) has 29.4 mL of oxygen sufficient for the surface oxidation of the formulation.

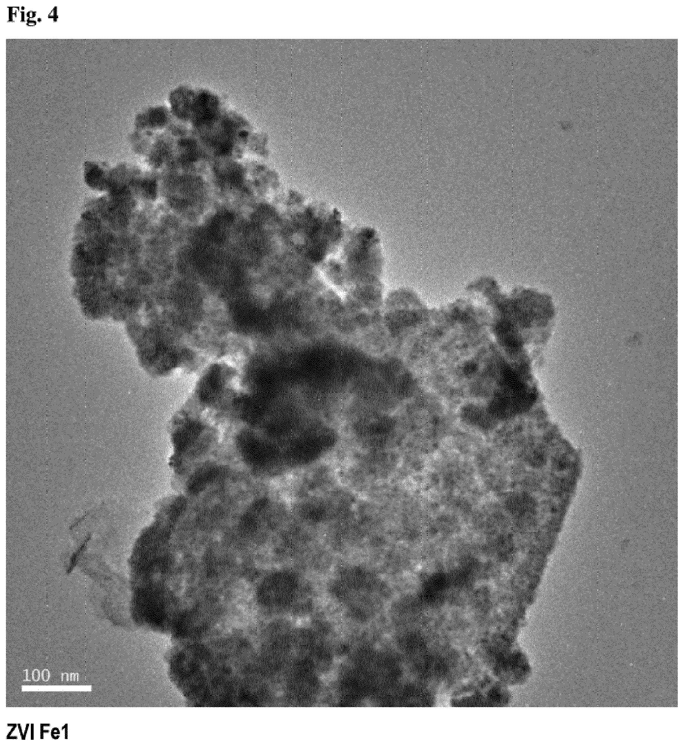

An electron microscopy study was carried out and it showed the structure of an aged ZVI nanoparticle of the core-oxide passivation coating type, as shown schematically in Fig. 3. It is confirmed by the electron microscope photograph of aged ZVI Fe1 illustrated in Fig. 4, in which brighter rings of iron oxides around a zero-valent iron grain are clearly seen.

The resulting ZVI formulation Fe1 was used to study antiviral activity after 6 months. Therefore, 100 mg ZVI was placed in 1 mL of PBS buffer (1X Phosphate Buffered Saline, pH 7.3-7.5; BioShop) for at least 36 hours. The formulation prepared in this way was used for antiviral properties studies of ZVI Fe1.

### Example 1b. ZVI formulation Fe2

A batch of the ZVI formulation as obtained in Example 1 was aged directly after freeze-drying through exposure to air at room temperature for 1 h until the color of the formulation changed from black to brown.

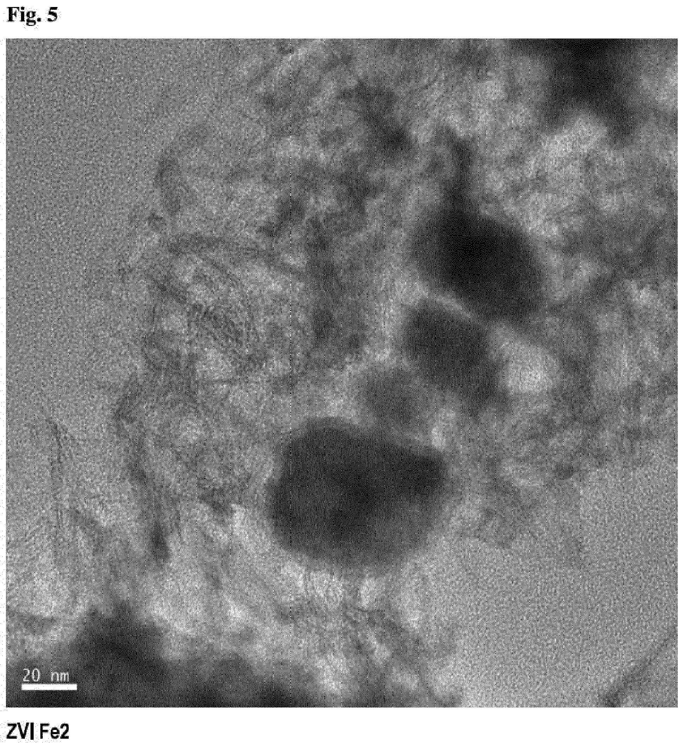

Aging conditions were selected as described in Example 1a. The structure of the resulting aged ZVI is illustrated in Fig. 5 by an electron microscope photograph.

The resulting ZVI formulation Fe2 was used to study antiviral activity after one week. Therefore, 100 mg ZVI was placed in 1 mL of PBS buffer (1X Phosphate Buffered Saline, pH 7.3-7.5; BioShop) for at least 36 hours. The formulation prepared in this way was used for antiviral properties studies of ZVI Fe2.

### Example 1c. ZVI formulation Fe3

A batch of the ZVI formulation as obtained in Example 1 was stored in argon after freeze-drying and aged directly before use through exposure to air at room temperature for 1 h until the color of the formulation changed from black to brown.

Aging conditions were selected as described in Example 1a.

Subsequently, 100 mg ZVI was placed in 1 mL of PBS buffer (1X Phosphate Buffered Saline, pH 7.3-7.5; BioShop) for at least 36 hours. The formulation prepared in this way was used for antiviral properties studies of ZVI Fe3.

### Example 1d. ZVI formulation Fe4

Preparation of ZVI in a reduction reaction of a mixture of iron(II) chloride and iron(II) sulfate with sodium borohydride.

The method was carried out in a glass flask having a volume of 5 dm³ fitted with a gas inlet and outlet. 40 g FeCl₂ and 40 g FeSO₄ x 4H₂O were dissolved in 1000 mL of degassed distilled water and 0.5 M iron salt solution was obtained. 2500 mL of 0.5 M (37.2 g in 1000 mL of degassed distilled water) NaBH₄ solution was prepared separately, and it was subsequently added dropwise at a rate of 0.25-0.3 mL/s to the iron salt solution until no further formation of black precipitate and release of hydrogen occurred, typically 2000 mL. The reaction was carried out in a protective atmosphere of inert argon gas. The reaction mixture was left to stand for 24 h, and the remaining 500 mL of the borohydride solution was added in portions (50 mL each) and left to stand for 24 h to stabilize the precipitate. After decanting, the precipitate was washed with distilled water until the Cl⁻ ions were completely removed. Subsequently, the precipitate was filtered using a Nylon fabric filter with a 20 DEN weave and dried by freeze-drying.

The resulting ZVI formulation was initially stored in a protective atmosphere of inert argon gas and subsequently was aged through exposure to air at room temperature for 1 h until the color of the formulation changed from black to brown. Aging conditions were selected as described in Example 1a.

The resulting ZVI formulation Fe4 was used to study antiviral activity after one week. Therefore, 100 mg ZVI was placed in 1 mL of PBS buffer (1X Phosphate Buffered Saline, pH 7.3-7.5; BioShop) for at least 36 hours. The formulation prepared in this way was used for antiviral properties studies of ZVI Fe4.

**Table 1. Characteristics of ZVI formulations Fe1 - Fe4**

| | Average particle size, Z-ave (nm) | Polydispersity index, PdI | d_{Intensity} (nm) | d_{Number} (nm) | Zeta potential (mV) |
|---|---|---|---|---|---|
| **Fe1** | 582.6 / 44.3 | 0.444 / 0.090 | 502.1 / 8.73 | 421.8 / 8.16 | 22.3 / 0.36 |
| **Fe2** | 402.3 / 14.8 | 0.506 / 0.056 | 439.3 / 42.0 | 144.5 / 95.78 | 28.8 / 3.41 |
| **Fe3** | 249.3 / 11.7 | 0.389 / 0.007 | 373.8 / 74.0 | 98.5 / 54.54 | 28.1 / 0.72 |
| **Fe4** | 889.0 / 58.4 | 0.380 / 0.058 | 763.3 / 39.3 | *551.0* / 82.01 | 16.1 / 0.36 |

Table 1 shows the characteristics of the obtained ZVI formulations Fe1 - Fe4, determined by the studies conducted which showed that the average particle size (hydrodynamic size) of the resulting formulations after freeze-drying was within a range of 250 to 900 nm. Electron microscopy studies of the formulations showed that they consisted of zero-valent iron nanoparticles with a size below 100 nm, forming clusters in the form of agglomerates.

Based on the conducted studies, it can be concluded that the aging time of the formulations affects the average hydrodynamic size of zero-valent iron particles which ranges from 250 nm for ZVI Fe3 (with the shortest aging time) and 580 nm for ZVI Fe1 (with the longest aging time).

Aging time had also an effect on the Zeta potential value of the studied formulations. The formulation obtained from a mixture of ferrous salts (ZVI Fe4) had the highest average hydrodynamic size of the particles and the lowest Zeta potential.

Such properties affect the activity of the resulting ZVI nanoparticles, as shown in the examples below.

### Example 2. Studying the antiviral activity of aged zero-valent iron (ZVI)

The antiviral activity of aged zero-valent iron was studied with representative A/California/04/2009 (H1N1) and A/PR8/1934 (H1N1) strains of influenza type A virus as examples.

**Example 2a.** ZVI Fe1 as obtained in Example 1a was subjected to an immunofluorescence assay to determine the amount of viral infectious units in one milliliter of the viral supernatant studied. The studies were carried out in concentrated solutions of the influenza type A virus (A/California/04/2009 (H1N1) or A/PR8/1934 (H1N1), having a titer of 1x10⁸ FFU/mL each). Therefore, 45 µL of virus solution was mixed with 5 µL of ZVI solution (having a concentration of 100 mg/mL) to obtain final ZVI concentrations of 0.75, 1.5, 3, 6 and 8 mg/mL (PBS buffer was used for dilution). The studied samples were incubated at 22°C for 2 hours. In addition, the antiviral efficacy of ZVI was also tested for a concentration of 3 mg/mL over 15, 30 and 60 minutes. In the subsequent stage, the supernatant containing the virus was collected using a magnetic stand, exploiting the superparamagnetic properties of ZVI.

In the subsequent stage, cells for an immunofluorescence assay were prepared. Madin-Darby canine kidney (MDCK) cells were seeded in 96-well plates in an amount of 20,000 cells in 100 µL per well in the 1x DMEM (Corning) medium supplemented with 10% fetal bovine serum (FBS; Gibco) and 1% PSG (penicillin - 100 U/mL, streptomycin - 100 mg/mL, L-glutamine- 2 mM; Gibco) and incubated for 24 hours in standard culture conditions (37°C, 5% CO₂, 95% humidity). Serial 10-fold dilutions of the studied viral supernatants were prepared in an infectious medium containing 0.3% BSA (Sigma), 100 U/mL penicillin, 100 mg/mL streptomycin (Sigma) in PBS buffer. The cells were washed twice with 100 µL of PBS buffer and infected with serial dilutions of viral supernatants (50 µL per well) for 1 hour at room temperature on a gently rocked platform. Subsequently, the infectious medium was removed and the DMEM post-infectious medium supplemented with 0.2% BSA, 67 U/mL penicillin, 67 mg/mL streptomycin, 1.3 mM glutamine, 2.5 µg/mL N-acetylated trypsin (Sigma), 1% Avicel was added and incubated for 18-26 hours (18 hours for the A/PR8/1934 virus; 26 hours for the A/California/04/2009 virus) at 33°C, 5% CO₂ and 95% humidity. The post-infectious medium was subsequently removed and the cells were fixed and permeabilized in PBS buffer containing 4% formaldehyde solution (Sigma), 0.5% Triton X-100 solution (BioShop) for 20 min at room temperature. Subsequently, the cells were washed twice with 100 µL of PBS buffer and incubated in 50 µL of blocking buffer containing 2% bovine serum albumin (Sigma) in PBS buffer at room temperature for 1 hour. The blocking buffer was subsequently replaced with 50 µL of a solution containing primary antibodies targeted to influenza virus nucleoproteins (MAB8257, Merck) diluted in PBS buffer (1 µg/mL) and incubated at room temperature for 1-2 hours. Subsequently, the cells were washed twice with 100 µL of PBS buffer. Then 50 µL of fluorescent-labeled secondary antibodies targeted to primary antibodies (AP160F, Merck) diluted in PBS buffer (6.6 µg/mL) were added and incubated at room temperature for 30-60 minutes. Infectious units of the influenza virus (focus forming units; FFU) were counted under a fluorescence microscope and the number of units in one milliliter of the supernatant was determined based on dilutions (FFU/mL).

It was found that when ZVI Fe1 having a concentration of 3 mg/mL was used, the viral titer significantly decreased as early as after 15 minutes of incubation with the influenza virus, from 7.33 log₁₀ to 6.02 log₁₀ for the A/PR8/1934 strain, which corresponded to 95.14% of the amount of the virus (Table 2, Fig. 2). Extension of the incubation time from 15 minutes to 120 minutes did not have a significant influence on an increase of the antiviral properties (p-value = 0.2535); it is therefore concluded that 15 minutes is the sufficient and at the same time minimum incubation time. In addition, already the lowest concentration of ZVI (i.e., 0.75 mg/mL) caused a significant decrease in the influenza virus titer after two hours of incubation with the virus, both for the A/California/04/2009 and the A/PR8/1934 strain (Table 2, Fig. 1). Furthermore, it was found that with increasing concentration of ZVI, the efficacy of inactivation of the influenza virus increased. At the highest ZVI concentration used (8 mg/mL), the influenza virus titer for the A/PR8/1934 strain decreased from 7.27 log₁₀ to 4.13 log₁₀, which corresponded to as much as 99.93% of the amount of the virus (Table 2, Fig. 1). Similar results were obtained for the A/California/04/2009 strain, and at the highest ZVI concentration tested (8 mg/mL), the influenza virus titer decreased from 7.54 log₁₀ to 4.90 log₁₀, which corresponded to as much as 99.77% of the amount of the virus.

Based on the obtained results it was concluded that the use of 8 ml/mL concentration of ZVI with incubation for two hours achieved the highest antiviral efficacy.

**Table 2. Decrease of the influenza virus titer after incubation with ZVI Fe1.**

| **Studying the antiviral properties of ZVI Fe1 depending on:** | | | | | | | |
|---|---|---|---|---|---|---|---|
| incubation time with ZVI for the A/PR8/1934 influenza virus | | | | | | | |
| incubation time [min] | 0 | 15 | 30 | | 60 | | 120 |
| influenza virus titer [FFU/mL] | 2.3E+07 | 1.1E+06 | 1.3E+06 | | 6.6E+05 | | 6.4E+05 |
| influenza virus titer expressed in log₁₀ | 7.33 ± 0.22 | 6.02 ± 0.22 | 6.09 ± 0.14 | | 5.76 ± 0.32 | | 5.77 ± 0.24 |
| efficacy in relation to control [%] | - | 95.14% ± 2.07% | 94.48% ± 1.41% | | 97.17% ± 1.27% | | 97.24% ± 1.17% |
| test probability (*p*-value) | - | 0.0019 (**) | 0.0013 (**) | | 0.0021 (**) | | 0.0011 (**) |

| concentration of ZVI for the A/PR8/1934 influenza virus | | | | | | | |
|---|---|---|---|---|---|---|---|
| ZVI concentration [mg/mL] | 0 | 0.75 | 1.5 | 3 | | 6 | 8 |
| influenza virus titer [FFU/mL] | 1.9E+07 | 8.2E+05 | 3.6E+05 | 1.6E+05 | | 1.3E+05 | 1.4E+04 |
| results expressed in log₁₀ | 7.27 ± 0.12 | 5.78 ± 0.41 | 5.54 ± 0.17 | 5.10 ± 0.38 | | 5.02 ± 0.35 | 4.13 ± 0.11 |
| efficacy in relation to control [%] | | 95.68% ± 3.24% | 98.07% ± 0.54% | 99.14% ± 0.63% | | 99.29% ± 0.50% | 99.93% ± 0.01% |
| test probability (*p*-value) | - | 0.0039 (**) | 0.0001 (***) | 0.0007 (***) | | 0.0004 (***) | <0.0001 (****) |

| concentration of ZVI for the A/California/04/2009 influenza virus | | | | | | | |
|---|---|---|---|---|---|---|---|
| influenza virus titer [FFU/mL] | 3.5E+07 | 4.2E+05 | 2.7E+05 | 1.4E+05 | | 6.9E+04 | 8.0E+04 |
| results expressed in log₁₀ | 7.54 ± 0.11 | 5.60 ± 0.18 | 5.40 ± 0.20 | 5.08 ± 0.26 | | 4.83 ± 0.09 | 4.90 ± 0.04 |
| effectiveness in relation to control [%] | | 98.81% ± 0.36% | 99.23% ± 0.27% | 99.61% ± 0.21% | | 99.81% ± 0.03% | 99.77% ± 0.02% |
| test probability (*p*-value) | - | <0.0001 (****) | <0.0001 (****) | <0.0001 (****) | | <0.0001 (****) | <0.0001 (****) |

The number of influenza virus infectious units was determined using an immunofluorescence assay for A/California/04/2009 (H1N1) and A/PR8/1934 (H1N1) strains. The relationship of the incubation time with ZVI was tested for 3 mg/mL, and the relationship with the ZVI concentration was tested for 2 hours of incubation. Control was a sample without ZVI and containing only PBS buffer. The data are expressed as a mean ± standard deviation for three independent experiments, each performed in triplicate. Student's two-tailed distribution unpaired t-test was performed for statistical comparison (*p < 0.05, **p < 0.01, ***p < 0.001, ****p < 0.0001).

**Example 2b.** ZVI Fe2 as obtained in Example 1b was studied according to the procedures shown in Example 2a.

It was found that when ZVI Fe2 in a concentration of 3 mg/mL was used, the viral titer significantly decreased as early as after 15 minutes of incubation with the influenza virus, from 7.09 log₁₀ to 5.03 log₁₀ for the A/PR8/1934 strain, which corresponded to 98.94% of the amount of the virus (Table 3, Fig. 2). Extension of the incubation time from 15 minutes to 120 minutes did not have a significant influence on an increase of the antiviral properties (p-value = 0.2279); it is therefore concluded that 15 minutes is the sufficient and at the same time minimum incubation time. In addition, already the lowest concentration of ZVI (i.e., 0.75 mg/mL) caused a significant decrease in the influenza virus titer after two hours of incubation with the virus, both for the A/California/04/2009 and the A/PR8/1934 strain (Table 3, Fig. 1). Furthermore, it was found that with increasing concentration of ZVI, the efficacy of inactivation of the influenza virus increased. At the highest ZVI concentration used (8 mg/mL), the influenza virus titer for the A/PR8/1934 strain decreased from 7.30 log₁₀ to 5.01 log₁₀, which corresponded to as much as 99.54% of the amount of the virus (Table 3, Fig. 1). Similar results were obtained for the A/California/04/2009 strain, and at the highest ZVI concentration tested (8 mg/mL), the influenza virus titer decreased from 7.54 log₁₀ to 6.05 log₁₀, which corresponded to as much as 96.60% of the amount of the virus.

Based on the obtained results that it was concluded that the use of 8 ml/mL concentration of ZVI with incubation for two hours achieved the highest antiviral efficacy.

**Table 3. Decrease of the influenza virus titer after incubation with ZVI Fe2.**

| **Studying the antiviral properties of ZVI Fe2 depending on:** | | | | | | | | | | |
|---|---|---|---|---|---|---|---|---|---|---|
| incubation time with ZVI for the A/PR8/1934 influenza virus | | | | | | | | | | |
| incubation time [min] | 0 | | 15 | | 30 | | 60 | | 120 | |
| influenza virus titer [FFU/mL] | 1.3E+07 | | 1.3E+05 | | 9.2E+04 | | 1.1E+05 | | 5.1E+04 | |
| influenza virus titer expressed in log₁₀ | 7.09 ± 0.13 | | 5.03 ± 0.38 | | 4.92 ± 0.25 | | 4.99 ± 0.23 | | 4.61 ± 0.34 | |
| efficacy in relation to control [%] | | | 98.94% ± 0.59% | | 99.27% ± 0.34% | | 99.14% ± 0.31% | | 99.60% ± 0.28% | |
| test probability (*p*-value) | - | | 0.0009 (***) | | 0.0002 (***) | | 0.0002 (***) | | 0.0003 (***) | |

| concentration of ZVI for the A/PR8/1934 influenza virus | | | | | | | | | | |
|---|---|---|---|---|---|---|---|---|---|---|
| ZVI concentration [mg/mL] | 0 | 0.75 | | 1.5 | | 3 | | 6 | | 8 |
| influenza virus titer [FFU/mL] | 2.2E+ 07 | 2.6E+06 | | 1.5E+06 | | 6.4E+05 | | 2.9E+05 | | 1.0E+05 |
| results expressed in log₁₀ | 7.30 ± 0.24 | 6.27 ± 0.41 | | 6.11 ± 0.28 | | 5.55 ± 0.60 | | 5.28 ± 0.47 | | 5.01 ± 0.02 |
| efficacy in relation to control [%] | - | 88.50% ± 9.48% | | 93.20% ± 3.89% | | 97.12% ± 2.86% | | 98.69% ± 1.20% | | 99.54% ± 0.01% |
| test probability (*p*-value) | - | 0.0204 (*) | | 0.0053 (**) | | 0.0094 (**) | | 0.0027 (**) | | <0.0001 (****) |

| concentration of ZVI for the A/California/04/2009 influenza virus | | | | | | | | | | |
|---|---|---|---|---|---|---|---|---|---|---|
| influenza virus titer [FFU/mL] | 3.5E+ 07 | 3.1E+06 | | 4.4E+06 | | 1.4E+06 | | 1.5E+06 | | 1.2E+06 |
| results expressed in log₁₀ | 7.54 ± 0.11 | 6.48 ± 0.11 | | 6.66 ± 0.33 | | 6.10 ± 0.30 | | 6.18 ± 0.11 | | 6.05 ± 0.20 |
| efficacy in relation to control [%] | | 91.19% ± 1.91% | | 87.42% ± 8.45% | | 95.96% ± 1.75% | | 95.66% ± 0.96% | | 96.60% ± 1.16% |
| test probability (*p*-value) | - | 0.0007 (***) | | 0.0004 (***) | | 0.0001 (***) | | 0.0001 (***) | | 0.0004 (***) |

The number of influenza virus infectious units was determined using an immunofluorescence assay for strains A/California/04/2009 (H1N1) and A/PR8/1934 (H1N1). The relationship of the incubation time with ZVI was tested for 3 mg/mL, and the relationship with the ZVI concentration was tested for 2 hours of incubation. Control was a sample without ZVI and containing only PBS buffer. The data are expressed as a mean ± standard deviation for three independent experiments, each performed in triplicate. Student's two-tailed distribution unpaired t-test was performed for statistical comparison (*p < 0.05, **p < 0.01, ***p < 0.001, ****p < 0.0001).

**Example 2c.** ZVI Fe3 as obtained in Example 1c was studied according to the procedures as shown in Example 2a.

It was found that when ZVI Fe3 having a concentration of 3 mg/mL was used, the viral titer significantly decreased as early as after 15 minutes of incubation with the influenza virus, from 7.24 log₁₀ to 5.41 log₁₀ for the A/PR8/1934 strain, which corresponded to 98.35% of the amount of the virus (Table 4, Fig. 2). However, the extension of the incubation time from 15 minutes to 120 minutes had a significant influence on an increase of the antiviral properties (p-value = 0.0326 (*)); it is therefore concluded that 120 minutes is the most effective incubation time. In addition, already the lowest concentration of ZVI (i.e., 0.75 mg/mL) caused a significant decrease in the influenza virus titer after two hours of incubation with the virus, both for the A/California/04/2009 and the A/PR8/1934 strain (Table 4, Fig. 1). Furthermore, it was found that with increasing concentration of ZVI, the efficacy of inactivation of the influenza virus increased. In addition, ZVI with a concentration of 3 mg/mL significantly decreased the viral titer as early as after 15 minutes of incubation with the influenza virus, from 7.24 log₁₀ to 5.41 log₁₀ for the A/PR8/1934 strain, which corresponded to 98.35% of the amount of the virus (Table 4, Fig. 2). At the highest ZVI concentration used (8 mg/mL), the influenza virus titer for the A/PR8/1934 strain decreased from 7.18 log₁₀ to 2.66 log₁₀, which corresponded to as much as 99.997% of the amount of the virus (Table 4, Fig. 1). Similar results were obtained for the A/California/04/2009 strain, and at the highest ZVI concentration tested (8 mg/mL), the influenza virus titer decreased from 7.54 log₁₀ to 5.44 log₁₀, which corresponded to as much as 99.20% of the amount of the virus. Based on the obtained results it was concluded that the used 8 ml/mL concentration of ZVI with incubation for two hours achieved the highest antiviral efficacy.

**Table 4. Decrease of the influenza virus titer after incubation with ZVI Fe3.**

| **Studying the antiviral properties of ZVI Fe3 depending on:** | | | | | | | | | | |
|---|---|---|---|---|---|---|---|---|---|---|
| incubation time with ZVI for the A/PR8/1934 influenza virus | | | | | | | | | | |
| incubation time with ZVI for the A/PR8/1934 influenza virus | 0 | | 15 | | 30 | | 60 | | 120 | |
| influenza virus titer [FFU/mL] | 1.8E+07 | | 3.0E+05 | | 2.3E+05 | | 8.2E+04 | | 5.5E+04 | |
| influenza virus titer expressed in log₁₀ | 7.24 ± 0.14 | | 5.41 ± 0.32 | | 5.36 ± 0.13 | | 4.91 ± 0.09 | | 4.71 ± 0.21 | |
| efficacy in relation to control [%] | - | | 98.35% ± 0.73% | | 98.70% ± 0.29% | | 99.54% ± 0.08% | | 99.70% ± 0.11% | |
| test probability (*p*-value) | - | | 0.0008 (***) | | <0.0001 (****) | | <0.0001 (****) | | <0.0001 (****) | |

| concentration of ZVI for the A/PR8/1934 influenza virus | | | | | | | | | | |
|---|---|---|---|---|---|---|---|---|---|---|
| ZVI concentration [mg/mL] | 0 | 0.75 | | 1.5 | | 3 | | 6 | | 8 |
| influenza virus titer [FFU/mL] | 1.5E+07 | 9.6E+05 | | 3.7E+05 | | 3.9E+04 | | 1.5E+04 | | 4.9E+02 |
| results expressed in log₁₀ | 7.18 ± 0.05 | 5.93 ± 0.25 | | 5.51 ± 0.28 | | 4.37 ± 0.60 | | 4.04 ± 0.46 | | 2.66 ± 0.21 |
| efficacy in relation to control [%] | - | 93.74% ± 2.84% | | 97.61% ± 1.16% | | 99.75% ± 0.20% | | 99.90% ± 0.06% | | 99.997% ± 0.001% |
| test probability (p-value) | - | 0.0011 (**) | | 0.0005 (***) | | 0.0012 (**) | | 0.0003 (***) | | <0.0001 (****) |

| concentration of ZVI for the A/California/04/2009 influenza virus | | | | | | | | | | |
|---|---|---|---|---|---|---|---|---|---|---|
| influenza virus titer [FFU/mL] | 3.5E+07 | 3.0E+06 | | 1.8E+06 | | 7.5E+05 | | 3.3E+05 | | 2.8E+05 |
| results expressed in log₁₀ | 7.54 ± 0.11 | 6.45 ± 0.14 | | 6.25 ± 0.03 | | 5.85 ± 0.18 | | 5.50 ± 0.15 | | 5.44 ± 0.13 |
| efficacy in relation to control [%] | - | 91.64% ± 2.26% | | 94.97% ± 0.24% | | 97.88% ± 0.65% | | 99.08% ± 0.23% | | 99.20% ± 0.18% |
| test probability (*p*-value) | - | 0.0005 (***) | | <0.0001 (****) | | 0.0002 (***) | | <0.0001 (****) | | <0.0001 (****) |

The number of influenza virus infectious units was determined using an immunofluorescence assay for strains A/California/04/2009 (H1N1) and A/PR8/1934 (H1N1). The relationship of the incubation time with ZVI was tested for 3 mg/mL, and the relationship with the ZVI concentration was tested for 2 hours of incubation. Control was a sample without ZVI and containing only PBS buffer. The data are expressed as a mean ± standard deviation for three independent experiments, each performed in triplicate. Student's two-tailed distribution unpaired t-test was performed for statistical comparison (*p < 0.05, **p < 0.01, ***p < 0.001, ****p < 0.0001).

**Example 2d.** ZVI Fe4 as obtained in Example 1d was studied according to the procedures as shown in Example 2a.

It was found that when ZVI Fe3 having a concentration of 3 mg/mL was used, the viral titer significantly decreased as early as after 15 minutes of incubation with the influenza virus, from 7.12 log₁₀ to 5.89 log₁₀ for the A/PR8/1934 strain, which corresponded to 94.10% of the amount of the virus (Table 5, Fig. 2). Extension of the incubation time from 15 minutes to 120 minutes did not have a significant influence on an increase of the antiviral properties (*p*-value = 0.2081); it is therefore concluded that 15 minutes is the sufficient and at the same time minimum incubation time. In addition, already the lowest concentration of ZVI (i.e., 0.75 mg/mL) showed a significant decrease in the influenza virus titer after two hours of incubation with the virus, both for the A/California/04/2009 and the A/PR8/1934 strain (Table 5, Fig. 1). Furthermore, it was found that with increasing concentration of ZVI, the efficacy of inactivation of the influenza virus increased. In addition, at the highest ZVI concentration used (8 mg/mL), the influenza virus titer for the A/PR8/1934 strain decreased from 7.18 log₁₀ to 5.09 log₁₀, which corresponded to as much as 99.997% of the amount of the virus (Table 5, Fig. 1). Similar results were obtained for the A/California/04/2009 strain, and at the highest ZVI concentration tested (8 mg/mL), the influenza virus titer decreased from 7.54 log₁₀ to 6.12 log₁₀, which corresponded to as much as 96.04% of the amount of the virus.

Based on the obtained results it was concluded that the use of 8 ml/mL concentration of ZVI with incubation for two hours achieved the highest antiviral efficacy.

**Table 5. Decrease of the influenza virus titer after incubation with ZVI Fe4.**

| **Studying the antiviral properties of ZVI Fe4 depending on:** | | | | | | | |
|---|---|---|---|---|---|---|---|
| incubation time with ZVI for the A/PR8/1934 influenza virus | | | | | | | |
| incubation time [min] | 0 | 15 | 30 | 60 | | 120 | |
| influenza virus titer | 1.4E+07 | 8.0E+05 | 7.9E+05 | 4.5E+05 | | 4.6E+05 | |
| [FFU/mL] | | | | | | | |
| influenza virus titer expressed in log₁₀ | 7.12 ± 0.15 | 5.89 ± 0.11 | 5.85 ± 0.23 | 5.60 ± 0.26 | | 5.59 ± 0.33 | |
| efficacy in relation to control [%] | - | 94.10% ± 1.20% | 94.18% ± 2.69% | 96.66% ± 1.81% | | 96.58% ± 1.69% | |
| test probability (*p-*value) | - | 0.0004 (***) | 0.0014 (**) | 0.001 (**) | | 0.0019 (**) | |

| concentration of ZVI for the A/PR8/1934 influenza virus | | | | | | | |
|---|---|---|---|---|---|---|---|
| ZVI concentration [mg/mL] | 0 | 0.75 | 1.5 | 3 | 6 | | 8 |
| influenza virus titer [FFU/mL] | 1.5E+07 | 1.4E+06 | 7.6E+05 | 4.6E+05 | 1.3E+05 | | 1.6E+05 |
| results expressed in log₁₀ | 7.18 ± 0.05 | 6.10 ± 0.22 | 5.80 ± 0.34 | 5.59 ± 0.33 | 5.09 ± 0.23 | | 5.09 ± 0.36 |
| efficacy in relation to control [%] | | 91.01% ± 4.10% | 95.01% ± 2.84% | 96.97% ± 1.51% | 99.13% ± 0.31% | | 98.99% ± 0.70% |
| test probability (*p-*value) | | 0.0012 (**) | 0.0021 (**) | 0.0012 (**) | 0.0001 (***) | | 0.0006 (***) |

| concentration of ZVI for the A/California/04/2009 influenza virus | | | | | | | |
|---|---|---|---|---|---|---|---|
| influenza virus titer [FFU/mL] | 3.5E+07 | 1.0E+07 | 4.3E+06 | 1.9E+06 | 1.6E+06 | | 1.4E+06 |
| results expressed in log₁₀ | 7.54 ± 0.11 | 6.95 ± 0.24 | 6.58 ± 0.26 | 6.26 ± 0.17 | 6.20 ± 0.11 | | 6.12 ± 0.19 |
| efficacy in relation to control [%] | - | 71.82% ± 13.58% | 87.92% ± 4.85% | 94.53% ± 1.69% | 95.41% ± 0.88% | | 96.04% ± 1.26% |
| test probability (*p-*value) | - | 0.0014 (**) | 0.0044 (**) | 0.0004 (***) | 0.0001 (***) | | 0.0004 (***) |

The number of influenza virus infectious units was determined using an immunofluorescence assay for A/California/04/2009 (H1N1) and A/PR8/1934 (H1N1) Strains. The relationship of the incubation time with ZVI was tested for 3 mg/mL, and the relationship with the ZVI concentration was tested for 2 hours of incubation. Control was a sample without ZVI and containing only PBS buffer. The data are expressed as a mean ± standard deviation for three independent experiments, each performed in triplicate. Student's two-tailed distribution unpaired t-test was performed for statistical comparison (*p < 0.05, **p < 0.01, ***p < 0.001, ****p < 0.0001).

### Example 2e. ZVI formulation in PBS

### Effect of aging time in PBS buffer on the antiviral activity of ZVI.

Directly after freeze-drying, a batch of the ZVI formulation obtained in Example 1 was placed in a test tube containing PBS buffer (1X Phosphate Buffered Saline, pH 7.3-7.5; BioShop). Freshly prepared ZVI in PBS buffer was used for antiviral activity studies, after 36 hours of ZVI aging in PBS buffer and after 7 days of ZVI aging in PBS buffer.

The influenza virus titer was determined using a hemagglutination assay for A/California/04/2009 (H1N1) strain. The assay was performed after 2 hours of ZVI incubation with the concentrated influenza virus solution. The results are expressed as log₂ for dilutions of the concentrated virus solution. Control was a sample without ZVI and containing only PBS buffer.

The results of the assay performed are listed in Table 6 below.

**Table 6. Effect of ZVI incubation time in PBS buffer on antiviral activity.**

| | Influenza virus titer | | |
|---|---|---|---|
| ZVI concentration [mg/mL] | freshly prepared ZVI in PBS buffer [log₂] | 36 hours of ZVI incubation in PBS buffer [log₂] | 7 days of ZVI incubation in PBS buffer [log₂] |
| 0 | 3 | 3 | 3 |
| 1 | 2 | 1 | 1 |
| 1.75 | 1 | 0 | 0 |
| 2.5 | 0 | 0 | 0 |
| 3.5 | 0 | 0 | 0 |

The study results of the effect of ZVI aging time (incubation) in PBS buffer shown in Table 6 on its antiviral activity clearly indicate that partially oxidized ZVI formulations have considerably higher antiviral activity. At a concentration of 1 mg/mL, freshly prepared ZVI in PBS buffer results in the influenza virus titer decrease by 1 log₂, while ZVI after 36 h of aging in PBS buffer results in the influenza viral titer decrease by 2 log₂. The influenza virus titer decreases to zero when 1.75 mg/mL of the aged formulation is used for 36 h in PBS, while the same effect is achieved with 2.5 mg/mL of freshly prepared ZVI in PBS buffer.

### Example 2f. Oxidized ZVI formulation

A batch of the ZVI formulation obtained in Example 1 was placed in a glass jar directly after freeze-drying and exposed to air at room temperature for 48 h until its color changed to light brown and was subsequently used for antiviral activity studies using the methodology as in the previous examples.

As a result of the study carried out no antiviral activity of the oxidized ZVI formulation was found.

The study results presented above show an effect of aging (surface oxidation of ZVI formulations) on the antiviral activity of the formulation. However, complete oxidation of ZVI formulations (Example 2f) results in their inactivation, despite the literature reports on the antiviral activity of iron oxides synthesized especially for this purpose.

Based on the studies carried out and the results obtained it may be concluded that zero-valent iron (ZVI), in form of aged nanoparticles forming agglomerates, especially obtained using the method defined herein, shows significant antiviral activity already at the lowest concentration used (i.e., 0.75 mg/mL) and at the shortest incubation time tested (i.e., 15 minutes). Therefore, ZVI can be used as an effective antiviral agent, including as an agent for the prevention and/or control of viral infection, especially caused by a virus of the *Orthomyxoviridae* family, such as the influenza virus, in particular influenza type A virus, and possibly also against other enveloped viruses based on their structural similarity and the mechanism of antiviral activity.

### References

1. Wieczorek, K.; Szutkowska, B.; Kierzek, E. Anti-influenza strategies based on nanoparticle applications. Pathogens 2020, 9, 1-24, doi:10.3390/pathogens9121020.
2. Kumar, S.R.; Paulpandi, M.; Manivelraja, M.; Mangalaraj, D.; Viswanathan, C.; Kannan, S.; Ponpandian, N. An in vitro analysis of H1N1 viral inhibition using polymer coated superparamagnetic Fe3O4 nanoparticles. RSC Adv. 2014, 4, 13409-13418, doi: 10.1 039/c3ra4 7542e.
3. Qin, T.; Ma, R.; Yin, Y.; Miao, X.; Chen, S.; Fan, K.; Xi, J.; Liu, Q.; Gu, Y.; Yin, Y.; et al. Catalytic inactivation of influenza virus by iron oxide nanozyme. Theranostics 2019, 9, 6920-6935, doi: 10.7150/thno.35826.
4. Palika, A.; Armanious, A.; Rahimi, A.; et al. An antiviral trap made of protein nanofibrils and iron oxyhydroxide nanoparticles. Nat. Nanotechnol. 2021, doi:10.1038/s41565-021-00920-5.
5. Foltynowicz, Z.; Maranda, A.; Czajka, B.; Wachowski, L.; Sa 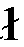 aciński, T. Efektywne usuwanie zanieczyszczeń pochodzenia organicznego i nieorganicznego za pomoc 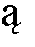 kompozytów na bazie nanocz 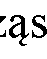 stek zero wartościowego zelaza n-Fe(0) [Effective removal of impurities of organic and inorganic origin using composites based on zero-valent iron n-Fe(0) nanoparticles]. *Mater. Wysokoenergetyczne* /*High Energy Mater.* **2018,** 108-146, doi:10.22211/matwys/0172.
6. Chiu, P.C. Applications of zero-valent iron (ZVI) and nanoscale ZVI to municipal and decentralized drinking water systems-A review. ACS Symp. Ser. 2013, 1123, 237249, doi:10.1021/bk-2013-1123.ch014.
7. Dong, H.; Li, L.; Lu, Y.; Cheng, Y.; Wang, Y.; Ning, Q.; Wang, B.; Zhang, L.; Zeng, G. Integration of nanoscale zero-valent iron and functional anaerobic bacteria for groundwater remediation: A review. Environ. Int. 2019, 124, 265-277, doi:10.1016/j.envint.2019.01.030.
8. Kar, S.; Tewari, P.K. Nanotechnology for domestic water purification; 2013; ISBN 9780857095442.
9. Fu, F.; Dionysiou, D.D.; Liu, H. The use of zero-valent iron for groundwater remediation and wastewater treatment: A review. J. Hazard. Mater. 2014, 267, 194-205, doi:10.1016/j.jhazmat.2013.12.062.
10. Shearer, A.E.H.; Kniel, K.E. Enhanced removal of norovirus surrogates, murine norovirus and tulane virus, from aqueous systems by zero-valent iron. J. Food Prot. 2018, 81, 1432-1438, doi:10.4315/0362-028XJFP-18-054.
11. Kim, J.Y.; Lee, C.; Love, D.C.; Sedlak, D.L.; Yoon, J.; Nelson, K.L. Inactivation of MS2 coliphage by ferrous ion and zero-valent iron nanoparticles. Environ. Sci. Technol. 2011, 45, 6978-6984, doi:10.1021/es201345y.
12. Shi, C.; Wei, J.; Jin, Y.; Kniel, K.E.; Chiu, P.C. Removal of viruses and bacteriophages from drinking water using zero-valent iron. Sep. Purif. Technol. 2012, 84, 72-78, doi:10.1016/j.seppur.2011.06.036.
13. You, Y.; Han, J.; Chiu, P.C.; Jin, Y. Removal and inactivation of waterborne viruses using zerovalent iron. Environ. Sci. Technol. 2005, 39, 9263-9269, doi:10.1021/es050829j.
14. Chopyk, J.; Kulkarni, P.; Nasko, D.J.; Bradshaw, R.; Kniel, K.E.; Chiu, P.; Sharma, M.; Sapkota, A.R. Zero-valent iron sand filtration reduces concentrations of virus-like particles and modifies virome community composition in reclaimed water used for agricultural irrigation. BMC Res. Notes 2019, 12, 1-8, doi:10.1186/s13104-019-4251- y.
15. Liu, Y.; Lowry, G. V. Effect of particle age (Fe0 content) and solution pH on NZVI reactivity: H2 evolution and TCE dechlorination. Environ. Sci. Technol. 2006, 40, 6085-6090, doi:10.1021/es060685o.
16. Phenrat, T.; Long, T.C.; Lowry, G. V.; Veronesi, B. Partial oxidation ("Aging") and surface modification decrease the toxicity of nanosized zerovalent Iron. Environ. Sci. Technol. 2009, 43, 195-200, doi:10.1021/es801955n.
17. Foltynowicz, Z. Nanoiron-Based Composite Oxygen Scavengers for Food Packaging. Compos. Mater. Food Packag. 2017, 209-234, doi:10.1002/9781119160243.ch6.
18. Glavee, G.N.; Klabunde, K.J.; Sorensen, C.M.; Hadjipanayis, G.C. Chemistry of borohydride reduction of iron(II) and iron(III) ions in aqueous and nonaqueous media. Inorg. Chem. 1995, 34, 28-35.
19. Woo, H.; Park, J.; Lee, S.; Lee, S. Effects of washing solution and drying condition on reactivity of nano-scale zero valent irons (nZVIs) synthesized by borohydride reduction. Chemosphere 2014, 97, 146-152, doi:10.1016/j.chemosphere.2013.11.010.
20. Goldstein, N.; Greenlee, L.F. Influence of synthesis parameters on iron nanoparticle size and zeta potential. J. Nanoparticle Res. 2012, 14, doi:10.1007/s11051- 012-0760-5.
21. Wang, Q.; Lee, S.; Choi, H. Aging study on the structure of Fe0-nanoparticles: Stabilization, characterization, and reactivity. J. Phys. Chem. C 2010, 114, 2027-2033, doi:10.1021/jp909137f.
22. Kim, H.S.; Kim, T.; Ahn, J.Y.; Hwang, K.Y.; Park, J.Y.; Lim, T.T.; Hwang, I. Aging characteristics and reactivity of two types of nanoscale zero-valent iron particles (FeBH and FeH2) in nitrate reduction. Chem. Eng. J. 2012, 197, 16-23, doi:10.1016/j.cej.2012.05.018.

## Claims

1. Non-therapeutic use of a zero-valent iron as an antiviral agent, preferably a disinfectant, wherein the zero-valent iron has a form of nanoparticles having sizes of <100 nm, which form agglomerates, wherein an average hydrodynamic size of nanoparticle agglomerates is within a range of 250 to 900 nm, wherein the zero-valent iron is subjected to aging by exposure of ZVI nanoparticles to air at room temperature, and wherein aging conditions are selected so that no more than 1% of the formulation thereof undergoes surface oxidation..

2. The non-therapeutic use according to claim 1, **characterized in that** the virus is a virus of the *Orthomyxoviridae* family, in particular the virus is an influenza virus, especially the influenza type A virus.

3. A method for the preparation of zero-valent iron obtained in a reduction reaction of an iron salt with sodium tetraborohydride NaBH₄, **characterized in that** an iron(II) salt or a mixture of iron(II) salts is used as a starting substance for the reduction in a molar ratio of the iron(II) salt to NaBH₄ of 1:2, the reaction is carried out in a protective atmosphere of an inert gas until precipitate forms, after precipitation the precipitate is subsequently decanted and washed until complete removal of anions of the salt used for the reduction and the precipitate is then filtered using a Nylon filter and dried.

4. The method according to claim 3, **characterized in that** iron(II) chloride or a mixture of iron(II) chloride and iron(II) sulfate is used as the starting substance.

5. The method according to claims 3 or 4, **characterized in that** a Nylon fabric filter is used, preferably with a 20 DEN weave.

6. The method according to any of claims 3-5, **characterized in that** the drying is carried out through freeze-drying.

7. The method according to any of claims 3-6, **characterized in that** the zero-valent iron after the drying step is subjected to aging, which aging is carried out until no more than 1% of the formulation is oxidized.

8. A zero-valent iron having a form of nanoparticles having sizes of <100 nm, which form agglomerates, wherein an average hydrodynamic size of nanoparticle agglomerates is within a range of 250 to 900 nm, for use as medicament in the prevention and/or control of viral infection, wherein the zero-valent iron is subjected to aging by exposure of ZVI nanoparticles to air at room temperature, and wherein aging conditions are selected so that no more than 1% of the formulation thereof undergoes surface oxidation.

9. The zero-valent iron for use according to claim 8, **characterized in that** the infection is caused by a virus of the *Orthomyxoviridae* family, in particular the virus is an influenza virus, especially the influenza type A virus.

## Patentansprüche

1. Nichttherapeutische Anwendung von nullwertigem Eisen als Virusbekämpfungsmittel, vorzugsweise als Desinfektionsmittel, wobei das nullwertige Eisen in Form von Nanopartikeln mit einer Größe von < 100 nm vorliegt, die Agglomerate bilden, wobei die hydrodynamische Durchschnittsgröße der Nanopartikel-Agglomerate im Bereich von 250 bis 900 nm liegt, wobei das nullwertige Eisen durch Exposition der ZVI-Nanopartikel gegenüber Luft bei Raumtemperatur gealtert wird und die Alterungsbedingungen so gewählt werden, dass nicht mehr als 1 % der Zubereitung einer Oberflächenoxidation unterliegt.

2. Nichttherapeutische Anwendung nach Anspruch 1, **dadurch gekennzeichnet, dass** es sich bei dem Virus um ein Virus aus der Familie der *Orthomyxoviridae* handelt, insbesondere um ein Influenzavirus und vor allem um ein Influenza-A-Virus.

3. Verfahren zur Herstellung von nullwertigem Eisen, das durch die Reduktion von Eisensalz mit Natriumtetraborat NaBH₄ gewonnen wird, **dadurch gekennzeichnet, dass** als Ausgangsstoff für die Reduktion Eisen(II)-Salz oder eine Mischung aus Eisen(II)-Salzen in einem Molverhältnis von Eisen(II)-Salz zu NaBH₄ von 1:2 verwendet wird, die Reaktion unter Schutzatmosphäre eines Inertgases bis zur Bildung eines Niederschlags durchgeführt wird, der Niederschlag nach der Ausfällung anschließend dekantiert und gewaschen wird, bis die Anionen des zur Reduktion verwendeten Salzes vollständig entfernt sind, und der Niederschlag anschließend unter Verwendung eines Nylonfilters filtriert und getrocknet wird.

4. Verfahren nach Anspruch 3, **dadurch gekennzeichnet, dass** als Ausgangsstoff Eisen(II)-chlorid oder eine Mischung aus Eisen(II)-chlorid und Eisen(II)-sulfat verwendet wird.

5. Verfahren nach Anspruch 3 oder 4, **dadurch gekennzeichnet, dass** ein Filter aus Nylongewebe, vorzugsweise mit einer Webart von 20 DEN, verwendet wird.

6. Verfahren nach einem der Ansprüche 3 bis 5, **dadurch gekennzeichnet, dass** die Trocknung durch Gefriertrocknung erfolgt.

7. Verfahren nach einem der Ansprüche 3 bis 6, **dadurch gekennzeichnet, dass** das nullwertige Eisen nach dem Trocknungsschritt gealtert wird, wobei diese Alterung so lange durchgeführt wird, bis nicht mehr als 1 % der Zubereitung einer Oxidation unterliegt.

8. Nullwertiges Eisen in Form von Nanopartikeln mit einer Größe von < 100 nm, die Agglomerate bilden, wobei die hydrodynamische Durchschnittsgröße der Nanopartikel-Agglomerate im Bereich von 250 bis 900 nm liegt, zur Anwendung als Arzneimitteln zur Vorbeugung und/oder Bekämpfung von Virusinfektionen, wobei das nullwertige Eisen durch Exposition der ZVI-Nanopartikel gegenüber Luft bei Raumtemperatur gealtert wird und die Alterungsbedingungen so gewählt werden, dass nicht mehr als 1 % der Zubereitung einer Oberflächenoxidation unterliegt.

9. Nullwertiges Eisen zur Anwendung nach Anspruch 8, **dadurch gekennzeichnet, dass** die Infektion durch ein Virus aus der Familie der *Orthomyxoviridae*, insbesondere durch ein Influenzavirus, vor allem durch ein Influenza-A-Virus, hervorgerufen wird.

## Revendications

1. Utilisation non thérapeutique du fer zérovalent en tant qu'agent antiviral, de préférence en tant qu'agent désinfectant, le fer zérovalent se présentant sous la forme de nanoparticules d'une taille inférieure à 100 nm qui forment des agglomérats, la taille hydrodynamique moyenne des agglomérats de nanoparticules se situant dans une plage comprise entre 250 et 900 nm, le fer zérovalent étant soumis à un vieillissement par exposition des nanoparticules de ZVI à l'air à température ambiante, les conditions de vieillissement étant choisies de manière à ce que pas plus de 1 % de la préparation ne subisse d'oxydation superficielle.

2. Application non thérapeutique selon la revendication 1, **caractérisée en ce que** le virus appartient à la famille des *Orthomyxoviridae*, en particulier **en ce qu'**il s'agit d'un virus de la grippe, et plus particulièrement d'un virus de la grippe de type A.

3. Procédé de fabrication de fer zérovalent, obtenu par réaction de réduction de sels de fer à l'aide de tétraborohydrure de sodium NaBH₄, **caractérisé en ce que** la substance de départ utilisée pour la réduction est un sel de fer(II) ou un mélange de sels de fer(II) dans un rapport molaire du sel de fer(II) au NaBH₄ égal à 1 :2, la réaction étant effectuée sous atmosphère protectrice d'un gaz inerte jusqu'à la formation d'un précipité ; une fois précipité, celui-ci est ensuite décanté et rincé jusqu'à l'élimination complète des anions du sel utilisé pour la réduction, puis filtré à l'aide d'un filtre en nylon et séché.

4. Procédé selon la revendication 3, **caractérisé en ce que** la substance de départ utilisée est du chlorure de fer(II) ou un mélange de chlorure de fer(II) et de sulfate de fer(II).

5. Procédé selon la revendication 3 ou 4, **caractérisé en ce que** l'on utilise un filtre en tissu de nylon, de préférence d'un tissage de 20 DEN.

6. Procédé selon l'une quelconque des revendications 3 à 5, **caractérisé en ce que** le séchage est effectué par lyophilisation.

7. Procédé selon l'une quelconque des revendications 3 à 6, **caractérisé en ce que**, après l'étape de séchage, le fer zérovalent est soumis à un vieillissement, ce dernier étant effectué jusqu'à ce que pas plus de 1 % de la préparation ne subisse d'oxydation.

8. Fer zérovalent sous forme de nanoparticules de taille < 100 nm, qui forment des agglomérats, la taille hydrodynamique moyenne des agglomérats de nanoparticules se situant dans une plage comprise entre 250 et 900 nm, destiné à être utilisé dans des médicaments pour la prévention et/ou le traitement d'infections virales, le fer zérovalent étant soumis à un vieillissement par exposition des nanoparticules de ZVI à l'air à température ambiante, les conditions de vieillissement étant choisies de telle sorte que pas plus de 1 % de la préparation ne subisse d'oxydation superficielle.

9. Fer zérovalent destiné à être utilisé conformément à la revendication 8, **caractérisé en ce que** l'infection est provoquée par un virus de la famille des *Orthomyxoviridae*, en particulier par un virus de la grippe, notamment par un virus de la grippe de type A.
